(19) **Europäisches Patentamt / European Patent Office / Office européen des brevets**

(11) **EP 4 593 025 A1**

(12) # EUROPEAN PATENT APPLICATION
published in accordance with Art. 153(4) EPC

(43) Date of publication:
**30.07.2025 Bulletin 2025/31**

(21) Application number: **23894842.6**

(22) Date of filing: **27.10.2023**

(51) International Patent Classification (IPC):
**G16H 30/40** (2018.01)  **G16H 30/20** (2018.01)
**G06V 10/82** (2022.01)  **G06V 10/75** (2022.01)
**A61B 5/055** (2006.01)  **A61B 6/03** (2006.01)
**G06N 3/08** (2023.01)

(52) Cooperative Patent Classification (CPC):
**A61B 5/055; A61B 6/03; G06N 3/08; G06V 10/75; G06V 10/82; G16H 30/20; G16H 30/40**

(86) International application number:
**PCT/KR2023/016871**

(87) International publication number:
**WO 2024/111913 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.11.2022 KR 20220158637**

(71) Applicant: **Polestar Healthcare Co., Ltd.
Seoul 06627 (KR)**

(72) Inventors:
• **YOON, Yeo Dong**
  **Seoul 06629 (KR)**
• **LEE, Eun Chong**
  **Yongin-si, Gyeonggi-do 16812 (KR)**

(74) Representative: **RGTH
Patentanwälte PartGmbB
Neuer Wall 10
20354 Hamburg (DE)**

(54) ## METHOD AND DEVICE FOR CONVERTING MEDICAL IMAGE USING ARTIFICIAL INTELLIGENCE

(57) One aspect of the present invention relates to a method for converting a medical image and, more specifically, to an image conversion method for converting a medical image using a generative adversarial neural network (GAN). One embodiment of the present invention has the effect of providing a medical image conversion method that enables medical staff to perform medical examination, diagnosis, and treatment with accurate information, and enables patients to receive appropriate medical services through conversion between medical images by a learning model that performs machine learning.

FIG. 1

FIRST OPERATION OF RECEIVING FIRST IMAGE SELECTED FROM PAIRED DATA SET INCLUDING FIRST AND SECOND IMAGES — S100

SECOND OPERATION OF CONSTRUCTING THIRD IMAGE BASED ON FIRST IMAGE, THIRD IMAGE MATCHING FIRST IMAGE AND BELONGING TO DIFFERENT DOMAIN THAN FIRST IMAGE — S110

THIRD OPERATION OF COMPARING THIRD IMAGE WITH SECOND IMAGE AND TRAINING LEARNING MODEL CONSIDERING RESULT OF COMPARISON — S120

FOURTH OPERATION OF COMPARING THIRD IMAGE WITH FIRST IMAGE AND TRAINING LEARNING MODEL CONSIDERING RESULT OF COMPARISON — S130

EP 4 593 025 A1

Description

## TECHNICAL FIELD

An aspect of the disclosure relates to a method of converting a medical image using artificial intelligence (AI) and, more particularly, to an image conversion method and device for converting a medical image using a generative adversarial network (GAN).

## BACKGROUND ART

The information disclosed in this section is only provided for an understanding of background information of embodiments of the disclosure and should not be taken as a description of the prior art.

In recent years, the conversion of medical images has emerged as an important issue in the field of radiology for medical professionals to diagnose and treat patients.

For the diagnosis and treatment of emergency room patients with brain hemorrhage, tumor patients, and the like, medical professionals may use non-enhanced (or non-contrast enhanced) CT images in which no contrast enhancers are used, enhanced (or contrast enhanced) CT images in which contrast enhancers are used, and the like.

Enhanced CT imaging is a type of medical imaging that uses a contrast agent to enhance tissue contrast to detect blood vessels, organs, cancerous tumors, and the like.

However, contrast agents may often cause side effects and even death due to cardiac arrest, shock death, or the like.

Because the side effects of contrast agents may not be predictable in advance, image conversion may help prevent these side effects and reduce the medical costs of contrast agents in a case in which non-enhanced CT or MR images may be converted to enhanced CT or MR images.

In addition, for individuals such as pregnant women who have MR images but no CT images due to unavailability of X-ray radiation or individuals who have CT images but are not MR imaged for reasons such as lack of time, cost, or implants, image conversion between imaging modalities may also help medical professionals to identify tissue boundaries or locate tumors without the use of contrast agents.

Furthermore, conversion between MR images, such as from a T1 image to a T2 image or from a T1 image to a diffusion image, may help medical professionals better understand lesions.

Recently, medical image conversion using artificial intelligence (AI) learning models has become an issue. For example, the learning model is trained to convert medical images, and after the training is completed, the learning model converts medical images as required by medical professionals.

Because these learning models are trained using a generative adversarial network (GAN), it is necessary to collect training data that forms paired data sets.

Here, a paired data set refers to a pair of training data which the learning model needs in the training. For example, to construct a synthetic MR image based on an original CT image, an original CT image and an original MR image matching the original CT image are required.

However, in the process of collecting training data, it may significantly difficult to obtain matching paired data sets due to temporal and spatial constraints. This is not only because patients may have limited access to medical imaging, but also because patients may not be able to undergo imaging with both CT and MR imaging devices at the same time.

This leads to problems such as the slice levels of input and target images not matching exactly.

Furthermore, in some cases, it is not possible to obtain a paired data set, such as in a case in which the input image is available but the target image is not.

Even in this case, it is urgent to present a learning model having a new learning method which enables the learning model to produce accurate output images.

The information disclosed in the Background section is technical information that the inventors possessed for, or acquired during, derivation of embodiments of the disclosure and should not be taken as known technology disclosed to the public before the filing of the embodiments of the disclosure.

## DISCLOSURE

### Technical Problem

Accordingly, an aspect of the disclosure has been made to solve the above-described problems, and an objective of the disclosure is to provide an artificial intelligence (AI) learning model which compares an output image with an input image and reflects a result to the learning model so that the output image accurately reflects the input image.

Another objective of the disclosure is to provide a method or device for converting a medical image based on a novel learning method which enables a learning model to generate an accurate output image in a case in which the input image and the target image do not match or in a case in which the input image is available but the target image is not available.

Another objective of the disclosure is to provide a method or device for converting a medical image which enables medical professionals to perform medical examination, diagnosis, and treatment with accurate information and patients to receive appropriate medical services.

The objectives of the disclosure are not limited to the foregoing description, and other objectives not explicitly disclosed herein will be clearly understood by a person having ordinary knowledge in the art to which the disclosure pertains from the description provided hereinafter.

**Technical Solution**

In order to achieve at least one of the above objectives, an aspect of the disclosure provides a method of converting a medical image using a generative adversarial network (GA), the method including:

a first operation of receiving a first image selected from a paired data set including the first image and a second image;
a second operation of constructing a third image based on the first image, the third image matching the first image and belonging to a different domain from the first image;
a third operation of comparing the third image with the second image and training a learning model considering a result of the comparison; and
a fourth operation of comparing the third image with the first image and training the learning model considering a result of the comparison.

Here, the fourth operation may include measuring image pattern similarity between the third image and the first image and feeding a value of the measurement back to the learning model.

In some embodiments, the image pattern similarity may include a Pearson correlation coefficient (PCC) or mutual information (MI).

The third operation may include calculating a match rate between the third image and the second image and feeding a value of the calculation back to the learning model, and the match rate is different from the image pattern similarity.

Another aspect of the disclosure provides an artificial intelligence (AI) learning model including: a learning part configured to receive a first image and construct a third image based on the first image;

a first comparator configured to generate a first comparison value by comparing the third image constructed by the learning part with a second image and feed the first comparison value back to the learning part; and
a second comparator configured to generate a second comparison value by comparing the third image constructed by the learning part with the first image and feed the second comparison value back to the learning part.

The first comparison value and the second comparison value may be generated in different manners.

The first comparator may compare a difference between the second image and the third image belonging to a single domain.

The first comparator may calculate a match rate between the second image and the third image and feeds a resulting value of the calculation back to the learning part.

The second comparator may compare a difference between the first image and the third image belonging to different domains.

The second comparator may calculate image pattern similarity between the first image and the third image and feeds a resulting value of the calculation back to the learning part.

Another aspect of the disclosure provides a device for converting a medical image using a generative adversarial network (GA), the device including at least one of:

a learning model configured to receive an input image and construct an output image matching the input image based on the input image; or a comparator configured to compare the output image with the input image and feed a value of the comparison back to the learning model.

The learning model may be trained by comparing the output image with the ground truth independently of the comparator.

The comparator may measure image pattern similarity between the input image and the output image and input a result of the measurement to the learning model.

The image pattern similarity may include a Pearson correlation coefficient (PCC) or mutual information (MI).

**Advantageous** Effects

According to an embodiment of the disclosure, an artificial intelligence (AI) learning model may compare an output image with an input image and reflect a result to the learning model so that the output image accurately reflects the input image.

According to another embodiment of the disclosure, a method or device for converting a medical image based on a novel learning method may enable the learning model to generate an accurate output image in a case in which the input image and the target image do not match or in a case in which the input image is available but the target image is not available.

According to another embodiment of the disclosure, a method or device for converting a medical image may enable medical professionals to perform medical examination, diagnosis, and treatment with accurate information and patients to receive appropriate medical services.

In addition, the disclosure has a variety of effects with excellent versatility according to embodiments, and such effects may be clearly understood from the following description of embodiments.

## DESCRIPTION OF DRAWINGS

The following drawings accompanying the specification illustrate embodiments of the disclosure and, together with the foregoing description of the disclosure, serve to provide further understanding of the technical spirit of the disclosure, and thus, the disclosure should not be construed as being limited to the drawings, wherein:

FIG. 1 illustrates a method of converting a medical image according to an embodiment of the disclosure.
FIG. 2 illustrates an AI learning model according to an embodiment of the disclosure.
FIG. 3 illustrates an embodiment of calculating the match rate between an output image and a target image.
FIG. 4 illustrates an AI learning model according to another embodiment of the disclosure.
FIG. 5 illustrates resulting values of measurement of image pattern similarity according to an embodiment of the disclosure.

## BEST MODE

Advantages and features of the disclosure, as well as methods of realizing the same, will be more clearly understood from the following detailed description of embodiments when taken in conjunction with the accompanying drawings. However, the disclosure is not limited to specific embodiments to be described hereinafter but should be understood as including a variety of modifications, equivalents, and alternatives within the spirit and scope of the disclosure. Rather, these embodiments are provided so that the description of the disclosure will be complete and will fully convey the scope of the disclosure to a person having ordinary skill in the art in the technical field to which the disclosure pertains. In the following description of the disclosure, a detailed description of related known technology will be omitted when the description may render the subject matter of the disclosure unclear.

The terminology used herein is for the purpose of describing particular embodiments only and is not intended to be limiting. As used herein, the singular forms are intended to include the plural forms as well, unless the context clearly indicates otherwise.

Terms, such as "comprise/include" or "have," or the like, as used herein, indicate that a feature, a number, a step, an operation, a component, a part or a combination thereof described in the disclosure is present, but do not preclude the possibility of the presence or addition of one or more other features, numbers, steps, operations, components, parts or combinations thereof in advance. Although terms, such as "first", "second", or the like, may be used to describe various components, these components should not be conceived of as being limited by these terms. These terms are only used to distinguish a component from another component.

Hereinafter, embodiments according to the disclosure will be described in detail with reference to the accompanying drawings, in which identical or similar components are given the same reference numerals, and repeated descriptions thereof will be omitted.

As used herein, input pictures and input images may be substantially interchangeable. Similarly, output pictures and output images may be substantially interchangeable, and target pictures and target images may also be interchangeable.

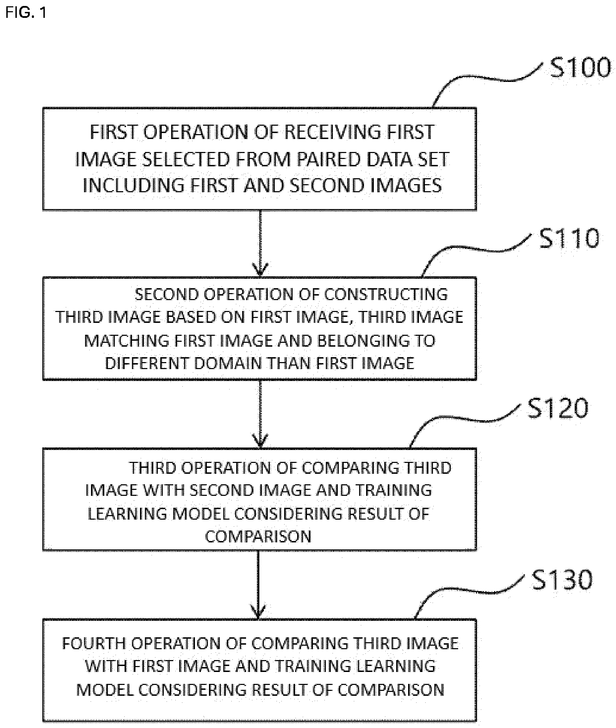

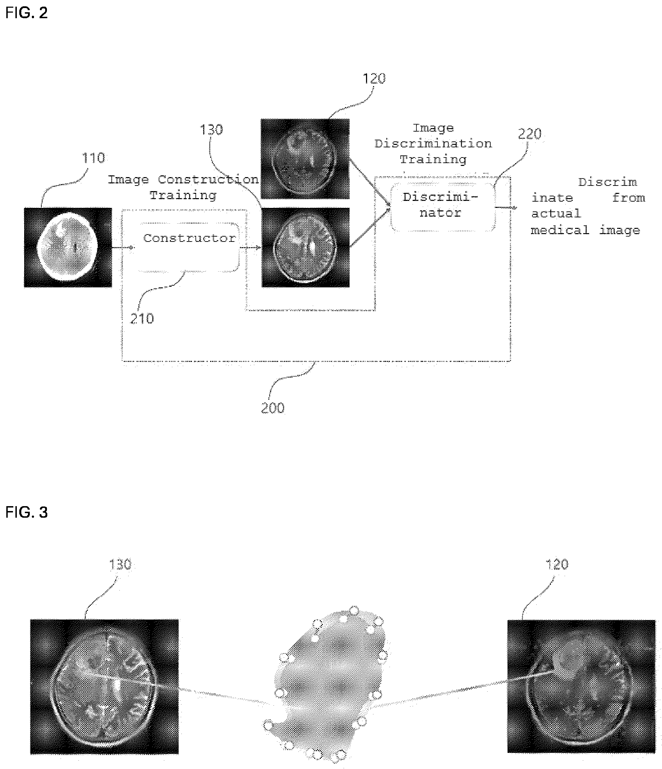

FIG. 1 illustrates a method of converting a medical image according to an embodiment of the disclosure, and FIG. 2 illustrates an artificial intelligence (AI) learning model according to an embodiment of the disclosure.

According to an embodiment of the disclosure, a method of converting a medical image using a generative adversarial network (GAN) may be provided.

The method of converting a medical image according to this embodiment may include: a first operation S100 of receiving a first image 110 selected from a paired data set including the first image 110 and a second image 120; a second operation S110 of constructing a third image 130 based on the first image 110, the third image 130 matching the first image 110 and belonging to a different domain from the first image 110;

a third operation S120 of comparing the third image 130 with the second image 120 and training the learning model considering a result of the comparison; and a fourth operation S130 of comparing the third image 130 with the first image 110 and training the learning model considering a result of the comparison.

The first image 110, the second image 120, and the third image 130 may be medical images. The medical images may be magnetic resonance images, such as 2D MR, 3D MR, 2D streaming MR, 4D MR, 4D volumetric MR, and 4D cine MR images; functional MR images, such as fMR, DCE-MR, and diffusion MR images; computed tomography (CT) images, such as 2D CT, cone beam CT, 3D CT, and 4D CT images; ultrasound images, such as 2D ultrasound, 3D ultrasound, and 4D ultrasound images; positron emission tomography (PET) images; X-ray images; fluoroscopic images; radiotherapy portal images; single-photon emission computed tomography (SPECT) images; computer generated synthetic images, such as pseudo-CT images; and the like.

Furthermore, the medical images may include medical image data, such as a training image, a ground truth image, a contoured image, a dose image, and the like.

In some embodiments, the medical image may include an image acquired using an image acquisition device, a computer generated synthetic image, and the like. The image acquisition device may include, for example, an MR imaging device, a CT imaging device, a PET imaging device, an ultrasound imaging device, a fluoroscopic device, a SPECT imaging device, an integrated linear accelerator, an MR imaging device, and the like. Furthermore, the image acquisition device is not limited to the above-described examples and may include a variety of medical imaging devices within the scope of the technical idea for acquiring medical images of a patient.

A paired data set may include a set of training data including images of an object such as a particular part of a patient.

For example, a CT imaging device and an MR imaging device may be used to acquire a CT image and an MR image, respectively, of a particular part of a patient, such as the head, in which case the CT image and the MR image may form a paired data set.

In a case in which a non-enhanced (or non-contrast enhanced) CT image and an MR image are respectively acquired for a particular part of a patient, the non-enhanced CT image and the MR image may form a paired data set. In a case in which an enhanced (or contrast enhanced) CT image and an MR image are respectively acquired, the enhanced CT image and the MR image may form a paired data set.

In a case in which T1 and T2 images are acquired from a particular part of a patient, the T1 and T2 images may form a paired data set.

In this context, the particular part of a patient may mean the particular part of a single patient. In a case in which imaged parts are the same, the imaged parts may refer to particular parts of different patients.

For example, CT and MR images acquired from a particular part of a single patient may form a paired data set, and CT and MR images acquired from the same parts of different patients may also form a paired data set.

Different domains of two images mean that the domains are different, for example, in a case in which respective patterns contained in two matching images have different contrasts or two images have different intensity distributions or different patterns.

Furthermore, images acquired using different imaging devices belong to different domains. For example, because CT and MR images are acquired using a CT imaging device and an MR imaging device, respectively, both images belong to different domains. Similarly, there are domain differences between ultrasound and CT images, between ultrasound and MR images, and between MR and PET images.

Furthermore, non-enhanced and enhanced images have different intensity distributions for the same part, and thus belong to different domains.

For MR images, T1 and T2 images are the same MR images, but have different intensity distributions and therefore belong to different domains. That is, in a case in which two images realized by reconstruction based on signals provided from an object have a difference in signals, sequences, or image realization processes on which the image realization is based, the two images having the difference belong to different domains. Furthermore, the medical image examples described above belong to different domains.

To illustrate the method of converting a medical image according to an embodiment of the disclosure, a case in which the image acquisition device has obtained a paired data set by acquiring a first image 110 and a second image 120 from the same part of a single patient will be described as an example. A case in which the first image 110 is an original CT image, the second image 120 is an original MR image, and the third image 130 is a synthetic MR image according to embodiments will be described.

The first operation S100 may include receiving, by the learning model, the first image 110 selected from the paired data set including the first image 110 and the second image 120. The first image 110 may include an input image processed from an original CT image with training data.

In the paired data set including the first image 110 and the second image 120, which one of the first image 110 and the second image 120 is to be selected as the input image may be determined by the manufacturer of medical device software which performs the method of converting a medical image according to this embodiment. In this case, which image is to be selected may be selected automatically by a selection algorithm, or may be selected manually by an operator.

The manufacturer may acquire the original CT image from the CT imaging device and the original MR image from the MR imaging device to form a paired data set, and may select the original CT image as the input image from the paired data set by an algorithm or manually.

The second operation S100 may include constructing the third image 130 based on the first image 110, in which the learning model having received the first image 110 constructs the third image 130 which matches the first image 110 and belongs to a different domain from the first image 110.

The learning model receives the original CT image as an input image and constructs a synthetic MR image based on the original CT image.

The process of the learning model constructing the third image 130 from the first image 110 which belongs to a different domain from the first image 110 may be performed by the following two processes according to embodiments. However, the process of the learning model constructing the third image 130 is not limited to the following two processes.

The first process may include one or more layers which receive the first image 110 obtained from the image acquisition device and perform convolutional operations to extract high-dimensional features having a smaller size than the first image 110, in which the layers may be represented by an artificial neural network configured to extract features. Such a feature extraction artificial neural network may receive two- or three-dimensional real image data obtained from the image acquisition device and perform convolutional operations to extract image features from the original images.

Describing this process by way of example, a convolutional layer which extracts the features of an image through a filter and a pooling layer which enhances the features and reduces the size of the image may be provided to extract the features of an image by repeating the convolution and the pooling.

In some embodiments, a convolutional layer including a plurality of layers may extract features of an input image given as an input from a first convolutional layer and produce a feature map from the features, a pooling layer may receive this feature map and produce a result having enhanced features and a reduced image size, and the output of the pooling layer may be input again into a second convolutional layer.

The second process includes one or more layers performing a deconvolution operation for the purpose of generating image data of different modalities from the output of the first process described above, in which these layers may be represented by an artificial neural network for generating images of different modalities. The artificial neural network which generates images of different modalities may perform a deconvolution operation on the result of the convolution operation of extracting the features by the artificial neural network in the first process to generate two- or three-dimensional image data of different modalities.

The third operation S120 may include operations of comparing the third image 130 with the second image 120 and training the learning model considering the comparison result. In a case in which the original CT image in the paired data set which includes the original CT image and the original MR image is determined to be the input image, the original MR image may be determined to be the target image. The second image 120 may correspond to ground truth. After constructing the third image 130, the learning model may be trained to reduce the difference between the constructed third image 130 and the second image 120 by comparing the constructed third image 130 with the second image 120. That is, the learning model may be trained to compare the synthetic MR image with the original MR image and continuously review whether the synthetic MR image is constructed to be similar to the original MR image, so that the synthetic MR image and the original MR image are the same. In some embodiments, an operation of calculating a loss value between the third image 130 and the second image 120 by applying a loss function to the algorithm may be included, and the parameters of the learning model may be updated based on the loss value.

In some embodiments, in the operation of calculating a loss value, the calculation of the loss value to update the learning model may be performed at each iteration. That is, the learning model may calculate the loss value between the synthetic MR image and the original MR image at each iteration during the training.

In a case in which the iteration includes a first iteration section and a second iteration section which are sequential, in some embodiments, the learning model may recognize overfitting and terminate the training in a case in which the absolute value of the change between the first loss value calculated in the first iteration section and the second loss value calculated in the second iteration section is less than a reference value.

In a case in which the learning model is sufficiently trained for the medical image conversion, the training may no longer be performed and be completed. The model having completed the training is now able to generate medical images as third images 130 with respect to a plurality of first images 110 having different data distributions, the third images 130 belonging to different domains from the first images 110.

A learning model 200 according to an embodiment of the disclosure may use a GAN model, and may include a constructor and a discriminator. The constructor may be trained to construct images, and the discriminator may be trained to discriminate images.

Referring to FIG. 2, in the learning model 200 using the GAN model, in a case in which the original CT image 110 is input as an input image to a constructor 210, the constructor 210 may be trained to construct a synthetic MR image 130 by the convolutional operation described above, and a discriminator 220 may be trained to discriminate the synthetic MR image 130 from an original MR image 120, i.e., a real medical image.

In some embodiments, the learning model 200 according to this embodiment may use a cycle GAN model (not shown). In this case, the learning model 200 may include the first constructor 210, the first discriminator 220, a second constructor, and a second discriminator. In a case in which the original CT image 110 is input to the first constructor 210 as an input image, the first constructor 210 may be trained to construct the synthetic MR image 130 through the convolutional operation described above, and the first discriminator 220 may be trained to discriminate a synthetic MR image from the original MR image 120, i.e., the real medical image. In a case in which the synthetic MR image 130 constructed by the first constructor 210 is then input to the second constructor, the second constructor may be trained to construct a synthetic CT image based on the synthetic MR image 130, and the second discriminator may be trained to discriminate a synthetic CT image from an original CT image.

Here, the original CT image 110 may correspond to the first image 110 in the method of converting a medical image described above, the synthetic MR image 130 may correspond to the third image 130, and the original MR image 120 may correspond to the second image 120.

In some embodiments, the third operation S120 may include calculating a match rate between the third image 130 and the second image 120 and feeding the calculated value back to the learning model. Note that the match rate is different from the image pattern similarity described above. Here, calculating the match rate is a method for determining the degree of difference between two images belonging to the same domain, and thus is different from the image pattern similarity described above. The third operation S120 and the fourth operation S130 may each compare the different comparison targets with different comparison methods and feed results back to the learning model.

FIG. 3 illustrates an embodiment of calculating the match rate between an output image and a target image.

In some embodiments, in a case in which a lesion region is marked on each of the same parts of a synthetic MR image and an original MR image, the calculation of the match rate may be performed by comparing the sizes of the lesion regions. For example, the match rate may be calculated by marking the location of a tumor or other lesion in the synthetic MR image and the original MR image, respectively, followed by conversion to a binary image.

In this case, the match rate may be calculated using an evaluation metric, such as the Hausdorff distance and the Dice similarity coefficient, or calculated quantitatively based on the difference between the centers of mass of the two lesion locations. Furthermore, in a case in which the target image and the output image are CT images, the match rate of the two images may be determined by comparing the radiation dose calculations for the lesions. In some embodiments, the determination may be performed by representing the difference between the synthetic MR image and the original MR image using numerical values such as MAE, RMSE, SSIM, or PSNR (hereinafter referred to as a performance metric).

Referring to FIG. 3, the process of calculating the match rate by aligning the lesion regions of the synthetic MR image 130, i.e., the output image, and the original MR image 120, i.e., the target image, and comparing the contours of the respective lesions using the Hausdorff distance measure is illustrated.

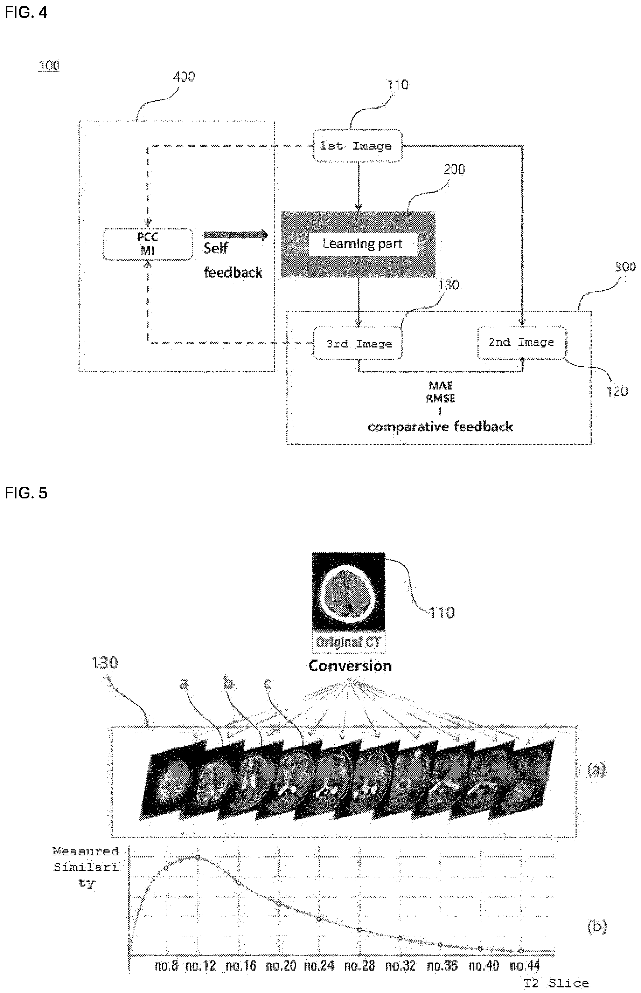

FIG. 4 illustrates an AI learning model according to another embodiment of the disclosure.

The fourth operation S130 may include comparing the third image 130 with the first image 110 and training the learning model considering the result of the comparison. Regarding the third image 130, the learning model receives the input image 110 and produces the output image 130 from the input image 110 by medical image conversion.

In a case in which the learning model outputs a synthetic MR image 130 which belongs to a different domain from the domain of the original CT image 110 based on the original CT image 110, the output image 130 is required to reflect the original CT image 110.

The objective of the medical image conversion according to the disclosure is to generate the output image 130 which accurately reflects the input image 110. However, there are cases in which the output image 130 does not accurately reflect the original CT image 110 because the learning model 200 is trained in the training process to compare the output image 130 with the target image 120 and reduce the difference. That is, there is a problem that the output image 130 may be inaccurate.

To address these issues, the method of converting a medical image according to an embodiment of the disclosure may perform a process of comparing the third image 130 with the first image 110 and feeding the result of the comparison back to the learning model. That is, the input image 110 and the output image 130 are compared to measure the degree to which the output image 130 reflects the input image, and the result is input again to the training process of the learning model 200.

In the fourth operation S130, the third image 130 and the first image 110 to be compared are images of different domains. That is, because the third image 130 is an MR image and the first image 110 is a CT image, the two images belong to different domains. This is in contrast to the third image 130 and the second image 120 belonging to the same domain, which are compared in the third operation S120. Because the third operation S120 compares the synthetic MR image and the original MR image, the images belonging to the same domain are compared. Therefore, the input value fed back to the learning model in the fourth operation S130 and the input value fed back to the learning model in the third operation S120 are different in nature.

In some embodiments, the fourth operation S130 may measure the image pattern similarity between the third image 130 and the first image 110 and feed a value of the measurement back to the learning model. Due to the different domains of the synthetic MR image and the original CT image, even the same region or organ of interest may have different intensity patterns in the two images. For example, in a case in which a particular region A may appear as a white area in the CT image, the same region may appear as a black area in the MR image. Therefore, to compare the differences between the two images of different domains, the image pattern similarity between the two images may be measured and the measurement may be fed back to the learning model to update the parameters of the learning model.

In some embodiments, one of various methods for measuring image pattern similarity is to use the Pearson correlation coefficient (PCC). In another example, mutual information (MI) may be used. However, these are only some of various methods for measuring image pattern similarity according to this embodiment, and a variety of other methods may be used.

Another embodiment of the disclosure may provide an AI learning model 100 including: a learning part 200 which receives a first image 110 and constructs a third image 130 based on the first image 110;

a first comparator 300 which compares the third image 130 constructed by the learning part 200 with a second image 120 to generate a first comparison value and feeds the first comparison value back to the learning part 200; and

a second comparator 400 which compares the third image 130 constructed by the learning part 200 with the first image 110 to generate a second comparison value and feeds the second comparison value back to the learning part 200.

In the AI learning model 100 according to this embodiment, the learning part 200 may receive the first image 110 as an input image and construct the third image 130 based on the first image 110. In some embodiments, the first image 110 and the third image 130 may include images belonging to the same domain or images belonging to different domains. In some embodiments, the first image 110 may refer to an input image, and the third image 130 may refer to an output image.

In a case in which the first image 110 and the third image 130 are images belonging to different domains, the AI learning model 100 according to this embodiment may implement the method of converting a medical image according to the above-described embodiments.

The AI learning model 100 according to this embodiment may include the first comparator 300 and the second comparator 400. The third image 130 and the second image 120 compared by the first comparator 300 may include images of the same modality. The third image 130 and the first image 110 compared by the second comparator 400 may include images of different modalities.

In some embodiments, the first comparator 300 may function to compare an output image and a target image and feed a first comparison value back to the learning part 200 so that the learning part 200 is trained by reflecting the first comparison value, and the second comparator 400 may function to compare the output image and an input image and feed a second comparison value back to the learning part 200 so that the learning part 200 is trained by reflecting the second comparison value.

The learning part 200 may be trained by receiving the first comparison value and the second comparison value, respectively. In some embodiments, the first comparison value and the second comparison value may be generated in different manners.

In some embodiments, the first comparator 300 may calculate the match rate between the second image 120 and the third image 130 and feeding a resulting value back to the learning part 200. For example, the first comparison value may include a result derived by calculating the match rate between images of same modality.

In some embodiments, the second comparator 400 may calculate image pattern similarity between the first image 110 and the third image 130 and feed a resulting value back to the learning part 200. For example, the second comparison value may include a result derived by calculating image pattern similarity between images of different modalities. The first comparator 300 may be configured to perform the process of the third operation S120 in the method of converting a medical image described above, and the second comparator 400 may be configured to perform the process of the fourth operation S130.

Formula 1 below is a mathematical formula for calculating mutual information (MI).

[Formula 1]

$$MI(X;Y) = \sum_{y \in Y} p(y) \sum_{x \in X} p(x|y) log \frac{p(x|y)}{p(x)}$$

Formula 1 is a mathematical formula for calculating the mutual information (MI) between two medical images X and Y. MI may be defined as an amount of information that indicates the relationship between information that the medical image X has and information that the medical image Y has.

In some embodiments, capitalized X and capitalized Y may refer to the original CT image 110 and the synthetic T2 image 130, respectively. The lowercase letter x may represent an intensity value for each pixel of the original CT image 110, and the lowercase letter y may represent an intensity value for each pixel of the synthetic T2 image 130.

In this case, the mutual information MI(X;Y) expressed by Formula 1 may represent the image pattern similarity between the original CT image 110 and the synthetic T2 image 130.

Specifically, Formula 1 may indicate the degree of correlation of the intensity distribution y of a particular region in the synthetic T2 image 130 with the intensity distribution x in the original CT image 110. For example, a bright area in the synthetic T2 image 130 may be dark in the original CT image 110, which may indicate how consistently these intensity distributions match.

In this embodiment, self-feedback for feeding back calculated values based on, e.g., mutual information (MI) representing image pattern similarity, may include feedback for ensuring that an output image constructed by the learning model sufficiently represents information in the input image.

In the process of acquiring a paired data set including input images and target images, even in a case in which the CT imaging device and the MR imaging device acquire the same part of a single patient, the input and target images do not completely match due to time constraints and differences in acquisition settings. In other words, in a case in which a patient is imaged using a CT imaging device and then MR imaged using an MR imaging device, the CT and MR images may be taken at different times and in different spaces, so it may not be possible to obtain CT and MR images that completely match.

In the self-feedback, in a case in which the slice of the input image and the slice of the target image in the paired data set do not perfectly match, the output image generated by the learning model is constructed based on the input image, rather than unconditionally following the target image.

In other words, in a case in which the input image and the target image do not exactly match, the learning model is trained so that information about, for example, tissues, foci, or the like shown in the input image is sufficiently represented in the output image of a different domain instead of being trained so that the output image is simply the same as the target image, but.

The self-feedback will be described in comparison with compassionate feedback.

The computational feedback is to feed the calculated match rate between the third image 130 and the second image 120 in the third operation S120 back to the learning model.

The self-feedback and the compositional feedback have different comparison targets for feedback. The self-feedback compares the input image with the output image, while the compositional feedback compares the output image with the target image. In other words, the self-feedback is to compare images belonging to different domains, while the comparative feedback is to compare images belonging to the same domain.

The comparative feedback simply reviews whether two images being compared have the same intensity patterns, whereas the self-feedback reviews the degree of similarity between the two images.

For example, because the original MR image and the synthetic MR image belong to the same domain of image information, the match rate between the two images may be quantified by a matrix operation between the constructed synthetic MR image and the original MR image and reflected to the learning model by the computational feedback.

However, in case of conversion from an original CT image to a synthetic MR image, information about the two images belongs to different image domains, and thus the similarity may not be quantified by a simple matrix operation between the images.

Because the original CT image and the synthetic MR image are images of different domains, a dark area in the CT image may be a bright area in the MR image or a dark area similar to the CT image. Therefore, a computational method other than a simple matrix operation must be used to reflect the similarity between the two images to the feedback process of the learning model.

The image pattern similarity will be described in more detail with reference to FIG. 5.

FIG. 5 illustrates resulting values obtained by measuring image pattern similarity according to an embodiment of the disclosure. Here, the calculation of image pattern similarity was performed using Formula 1. FIG. 5 (a) shows various output images output by the learning model after receiving an original CT image. Here, output images are synthetic T2 images. FIG. 5 (b) shows calculated image pattern similarity values of the output images.

FIG. 5 shows brain images. FIG. 5 shows numerical values of comparison of a single slice of the original CT image with the entire synthetic T2 slices. For the respective 8th, 12th, 20th, 24th, 28th, 32nd, 36th, 40th, and 44th slices of the original CT image, i.e., the input image, the learning model generated 8th, 12th, 20th, 24th, 28th, 32nd, 36th, 40th, and 44th slices as output T2 images to match the input image by comparing with a target image.

Here, the original CT image shown as the input image in FIG. 5 is the 12th slice image, and the 10 synthetic T2 images shown as the output images represent the 8th, 12th, 20th, 24th, 28th, 32nd, 36th, 40th, and 44th slices, respectively.

Referring to FIG. 5, it may be seen that the 12th slice image of the original CT image, i.e., the input image, has the highest value of measured similarity with the 12th slice image "a" of the output T2 image, i.e., the output image. It may also be seen that the 12th slice image of the original CT image, i.e., the input image, has a rather lower value of measured similarity with the 16th slice image "b" of the output T2 image or the 20th slice image "c" of the output T2 image than with the 12th slice image "a." Referring to FIG. 5, it may be seen that large ventricular structures appear in the 16th slice image "b" and 20th slice image "c" of the output T2 image, while no ventricular structure appears in the 12th slice of the input image. Only in the 12th slice of the output image, the ventricular structure is not visible as in the input image.

Accordingly, it may be seen that the 12th slices of the original CT image, i.e., the input image, and the synthetic T2 image, i.e., the output image, have high similarity and the remaining slices of the original CT and synthetic T2 images have low similarity, and the slicelevel match rate may be calculated based on pattern similarity.

In other words, the anatomy is most similar between the 12th slice of the original CT image and the 12th slice of the output synthetic T2 image.

The image pattern similarity is to compare the association of representations of anatomical structures between two images. Medical images show different intensity patterns depending on the image type, even for the same anatomical structure. Because the 16th slice image "b" and the 20th slice image "c" of the output image show the ventricular structure, which is not shown in the input image, the two slice images do not properly reflect the image pattern of the original image and thus show low similarity.

The disclosure enables a computer to verify whether an image generated by the above mathematical Formula 1 is properly converted based on the information about the original image and reflect the result of the verification to the AI learning model to solve problems such as artificially creating a structure that does not exist.

The image pattern similarity may mean, for example, similarity in the volumes of gray and white matters, the thicknesses of skulls, the diameters of skulls, and the like.

Even in a case in which the synthetic MR image generated by the learning model is the target image, the learning model generates an inaccurate synthetic MR image without slice matching between the original MR image, i.e., the target image, and the original CT image, i.e., the input image. To prevent this, the self-feedback is added.

That is, FIG. 5 shows that the output image produced by reflecting the target image that matches the level of the input image is more similar to the input image, and the output image produced by reflecting the target image that does not match the level of the input image is less similar to the input image.

In the case of the brain, matching between input and target images is not a significant issue because the brain is rigid due to fixation by the skull. However, in the case of the abdomen, the position and shape of the organs change significantly as the patient breathes. This means that it is difficult to perfectly match the CT and MR images because the internal organs are highly fluid depending on the condition.

Therefore, because the slice level of the target image sometimes does not exactly match the slice level of the input image even in a case in which the learning model is properly trained, it is necessary for the learning model to recognize through the self-feedback whether the output image constructed based on the input image properly expresses the texture of the input image.

FIG. 5 shows the plurality of T2 images as a result obtained by comparing with the target image and shows the degree of similarity to the original CT image.

The results indicate a high degree of similarity between the slice of the input image and the slice of the target image at the same level. Although FIG. 5 corresponds to the brain, the disclosure allows the conclusion that there is high similarity between the slice of the input image and the slice of the target image at the same level to be used for the abdomen or other organs that are difficult to match.

In other words, image similarity may not be used much for brain images, but there may be mismatches or no paired data at all for images of the abdomen, and the like. According to embodiments of the disclosure, even in such cases, the learning model may be updated by feeding the measurement of the image pattern similarity between the input image and the output image back to the learning model to solve the problem of low similarity of the output image caused by the mismatch or lack of paired data between the input image and the target image.

According to another embodiment of the disclosure, an image conversion device for converting a medical image using a generative adversarial network (GAN) may be provided.

The image conversion device includes: a learning model configured to receive an input image and construct an output image matching the input image based on the input image; and a comparator configured to compare the output image with the input image and feed a value of the comparison back to the learning model.

In the method of converting a medical image according to any of the above-described embodiments, the input image may be substantially the same as the first image 110, and the output image may be substantially the same as the third image 130.

In some embodiments, the learning model may be trained by comparing the output image and the ground truth independently of the comparator. The comparator may also be configured to compare the input image and the output image by measuring the image pattern similarity between the input image and the output image.

The image pattern similarity may include the Pearson correlation coefficient (PCC) or mutual information (MI).

For example, in this embodiment, the learning model may receive an original CT image as an input and construct a synthetic MR image from the original CT image using the GAN model. The comparator may compare the original CT image provided as an input to the learning model and the synthetic MR image produced by the learning model, measure the image pattern similarity between the original CT image and the synthetic MR image, and input the result to the learning model.

The above-described embodiments of the disclosure may be implemented in the form of computer programs executable on a computer using various components, and such computer programs may be stored in computer-readable media. Examples of the computer-readable media may include: magnetic media such as hard disks, floppy disks, and magnetic tapes; optical recording media such as CD-ROMs and DVDs; magneto-optical media such as floptical disks; and hardware devices such as ROMs, RAMs, and flash memories specifically configured to store program instructions and execute the program instructions.

Furthermore, the computer programs may be specifically designed and configured for the disclosure or may be known and available to a person having ordinary knowledge in the art of computer software. Examples of computer programs may include machine code produced by compilers and high-level language code executable on computers using interpreters.

In the specification (in particular Claims) of the disclosure, the use of the term "the" and similar denoting terms may correspond to both singular and plural forms. Furthermore, recitation of ranges of values herein are intended merely to refer to respective separate values falling within the respective ranges and, unless otherwise indicated herein, the respective separate values are incorporated herein as if individually recited herein.

Finally, the operations of any method described herein may be performed in any suitable order unless otherwise indicated herein or otherwise clearly contradicted by the context. However, the operations shall not be limited to the described sequence. The use of any examples or illustrative languages (e.g., "such as") provided herein, is intended merely to better illustrate the disclosure and does not pose a limitation on the scope of the disclosure unless otherwise defined by the Claims. Furthermore, a person having ordinary knowledge in the art will appreciate that various modifications, combinations, and changes are possible according to design conditions and factors within the scope of the Claims or equivalents thereof.

Therefore, the spirit of the disclosure shall not be limited to the above-described embodiments, and the entire scope of the appended claims and equivalents thereof will fall within the scope and spirit of the disclosure.

<Description of Reference Numerals of Drawings>

    100: AI learning model
    110: first image
    120: second image
    130: third image
    200: learning part
    210: constructor
    220: discriminator
    300: first comparator
    400: second comparator

**Claims**

1. A method of converting a medical image using a generative adversarial network (GA), the method comprising:

   a first operation of receiving a first image selected from a paired data set including the first image and a second image;
   a second operation of constructing a third image based on the first image, the third image matching the first image and belonging to a different domain from the first image;
   a third operation of comparing the third image with the second image and training a learning model considering a result of the comparison; and
   a fourth operation of comparing the third image with the first image and training the learning model considering a result of the comparison.

2. The method of claim 1, wherein the fourth operation comprises measuring image pattern similarity between the third image and the first image and feeding a value of the measurement back to the learning model.

3. The method of claim 2, wherein the image pattern similarity comprises a Pearson correlation coefficient (PCC) or mutual information (MI).

4. The method of claim 2, wherein the third operation comprises calculating a match rate between the third image and the second image and feeding a value of the calculation back to the learning model, and the match rate is different from the image pattern similarity.

5. An artificial intelligence learning model comprising:

a learning part configured to receive a first image and construct a third image based on the first image;
a first comparator configured to generate a first comparison value by comparing the third image constructed by the learning part with a second image and feed the first comparison value back to the learning part; and
a second comparator configured to generate a second comparison value by comparing the third image constructed by the learning part with the first image and feed the second comparison value back to the learning part.

6. The artificial intelligence learning model of claim 5, wherein the first comparison value and the second comparison value are generated in different manners.

7. The artificial intelligence learning model of claim 5, wherein the first comparator compares a difference between the second image and the third image belonging to a single domain.

8. The artificial intelligence learning model of claim 7, wherein the first comparator calculates a match rate between the second image and the third image and feeds a resulting value of the calculation back to the learning part.

9. The artificial intelligence learning model of claim 5, wherein the second comparator compares a difference between the first image and the third image belonging to different domains.

10. The artificial intelligence learning model of claim 9, wherein the second comparator calculates image pattern similarity between the first image and the third image and feeds a resulting value of the calculation back to the learning part.

11. A device for converting a medical image using a generative adversarial network (GA), the device comprising:

a learning model configured to receive an input image and construct an output image matching the input image based on the input image; and
a comparator configured to compare the output image with the input image and feed a value of the comparison back to the learning model.

12. The device of claim 11, wherein in a case in which there is ground truth, the learning model is trained by comparing the output image with the ground truth independently of the comparator.

13. The device of claim 11, wherein the comparator measures image pattern similarity between the input image and the output image and inputs a result of the measurement to the learning model.

14. The device of claim 13, wherein the image pattern similarity comprises a Pearson correlation coefficient (PCC) or mutual information (MI).

FIG. 1

S100

FIRST OPERATION OF RECEIVING FIRST
IMAGE SELECTED FROM PAIRED DATA SET
INCLUDING FIRST AND SECOND IMAGES

S110

SECOND OPERATION OF CONSTRUCTING
THIRD IMAGE BASED ON FIRST IMAGE, THIRD IMAGE
MATCHING FIRST IMAGE AND BELONGING TO
DIFFERENT DOMAIN THAN FIRST IMAGE

S120

THIRD OPERATION OF COMPARING THIRD
IMAGE WITH SECOND IMAGE AND TRAINING
LEARNING MODEL CONSIDERING RESULT OF
COMPARISON

S130

FOURTH OPERATION OF COMPARING THIRD IMAGE
WITH FIRST IMAGE AND TRAINING LEARNING
MODEL CONSIDERING RESULT OF COMPARISON

FIG. 2

FIG. 3

FIG. 4

FIG. 5

## INTERNATIONAL SEARCH REPORT

| | International application No. |
| --- | --- |
| | **PCT/KR2023/016871** |

### A. CLASSIFICATION OF SUBJECT MATTER

**G16H 30/40**(2018.01)i; **G16H 30/20**(2018.01)i; **G06V 10/82**(2022.01)i; **G06V 10/75**(2022.01)i; **A61B 5/055**(2006.01)i; **A61B 6/03**(2006.01)i; **G06N 3/08**(2006.01)i

According to International Patent Classification (IPC) or to both national classification and IPC

### B. FIELDS SEARCHED

Minimum documentation searched (classification system followed by classification symbols)

G16H 30/40(2018.01); A61B 5/00(2006.01); G06N 3/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생성적 적대 신경망(generative adversarial networks, GAN), 의료 영상(medical image), 변환(translation), 페어드 데이터 셋(paired data set), 도메인(domain), 학습(learning)

### C. DOCUMENTS CONSIDERED TO BE RELEVANT

| Category* | Citation of document, with indication, where appropriate, of the relevant passages | Relevant to claim No. |
| --- | --- | --- |
| Y | YANG, Guang et al. DAGAN: Deep De-Aliasing Generative Adversarial Networks for Fast Compressed Sensing MRI Reconstruction. IEEE Transactions on Medical Imaging. 21 December 2017, vol. 37, no. 6, pp. 1310-1321. <br> See pages 1310 and 1313-1316. | 1-14 |
| Y | DENCK, Jonas et al. MR-Contrast-Aware Image-to-Image Translations with Generative Adversarial Networks. arXiv. 03 April 2021, pp. 1-11. <br> See pages 1-4. | 1-14 |
| A | APPALARAJU, Srikar et al. Image similarity using Deep CNN and Curriculum Learning. arXiv. 13 July 2018, pp. 1-9. <br> See entire document. | 1-14 |
| A | AHMAD, Waqar et al. A new generative adversarial network for medical images super resolution. Scientific Reports. 09 June 2022, vol. 12, no. 9533, pp. 1-20. <br> See entire document. | 1-14 |

☑ Further documents are listed in the continuation of Box C.    ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **02 February 2024** | **02 February 2024** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/016871** |

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2022-0137215 A (KOREA UNIVERSITY RESEARCH AND BUSINESS FOUNDATION) 12 October 2022 (2022-10-12)<br>    See entire document. | 1-14 |
| PX | KR 10-2586483 B1 (PODEROSA INC.) 10 October 2023 (2023-10-10)<br>    See entire document.<br>    * This document is a published earlier application that serves as a basis for claiming priority of the present international application. | 1-14 |

Form PCT/ISA/210 (second sheet) (July 2022)

**INTERNATIONAL SEARCH REPORT**
Information on patent family members

| International application No. |
| --- |
| **PCT/KR2023/016871** |

| Patent document cited in search report | Publication date (day/month/year) | Patent family member(s) | Publication date (day/month/year) |
| --- | --- | --- | --- |
| KR 10-2022-0137215 A | 12 October 2022 | None | |
| KR 10-2586483 B1 | 10 October 2023 | None | |

Form PCT/ISA/210 (patent family annex) (July 2022)